# EUROPEAN PATENT APPLICATION

(11) **EP 4 806 499 A1**
(43) Date of publication of application: **16.09.2026**
(21) Application number: 26195645.2
(22) Date of filing: 21.06.2022
(51) Int. Cl.: A61N 1/04, A61B 5/296, A61N 1/36

(54) **MULTI-ELECTRODE PAD FOR TRANSCUTANEOUS STIMULATION**

(30) Priority: 22.06.2021 US 202163213299 P; 22.06.2021 US 202163213400 P
(62) Document number(s) of the earlier application(s) in accordance with Art. 76 EPC: 22829095.3 / 4 359 061
(71) Applicant: Niche Biomedical, Inc., Los Angeles, CA 90024 (US)
(72) Inventor: The designation of the inventor has not yet been filed
(74) Representative: Marks & Clerk LLP

(57) **Abstract**

Systems and methods that can employ and/or produce an improved multi-electrode pad configured to adhere to a patient's skin and provide transcutaneous stimulation to a portion of tissue are described. The electrode pad can include a flexible substrate. A plurality of electrodes can be arranged in an array on or within the flexible substrate. Each of the plurality of electrodes is configured to apply a stimulation waveform. Conductive traces are applied to the flexible substrate and coupled to each of the plurality of electrodes such that each of the plurality of electrodes is independently addressable through a single external cable.

## Description

### CROSS-REFERENCE TO RELATED APPLICATIONS

This application claims the benefit of U.S. Provisional Patent Application Nos. 63/213,299 and 63/213,400, each flied June 22, 2021, each entitled MULTI-ELECTRODE PAD FOR TRANSCUTANEOUS STIMULATION. The subject matter of these applications is incorporated herein by reference in their entirety.

### Technical Field

The present disclosure relates generally to transcutaneous stimulation and, more specifically, to systems and methods that can employ an improved multi-electrode pad for transcutaneous stimulation, as well as methods for manufacturing the improved multi-electrode pad.

### Background

Skin surface electrodes are commonly used in medicine to provide transcutaneous stimulation. One example of transcutaneous stimulation is external defibrillation, which uses two or more skin electrodes to deliver high voltages to the heart for the restoration of normal rhythms. In addition, transcutaneous stimulation via skin surface electrodes may be used to activate muscles for strengthening or rehabilitation, to modulate nerve signals to block pain or provide other functional improvement, to facilitate the repair and regrowth of a damaged nerve, and/or to up-regulate or down-regulate brain regions for applications including seizure prevention, tremor suppression, or treatment of conditions such as depression, stroke, traumatic brain injury, or the like.

Commercially-marketed skin surface electrodes are not designed based on any physiological feature, electrical property, or specific therapeutic end-use. Additionally, commercially marketed skin electrodes generally have only a single active electrode site, with one cable required per electrode to apply stimulation, which can lead to a mess of cables when a plurality of electrodes are need for a therapy, which may even be hazardous. Furthermore, when using individual electrodes, it is difficult to repeatedly maintain precise positions and orientations of electrodes when a plurality is needed over multiple days and different patients. In practice, most skin surface electrodes are made of a fabric backing layer, a conductive middle layer connected to a lead and a bottom hydrogel layer for interfacing with the skin. Other electrode designs (e.g., with stainless steel directly contacting the skin or dry conductive carbon) have been proposed but are disfavored due to their higher impedances that limit high-current stimulation. Electrode arrays with multiple electrodes on the same substrate have been developed but are bulky, heavy, and require a strap or large amounts of tape to hold to the skin. Additionally, these electrode arrays are unable to stimulate from all electrodes in the array independently and simultaneously, include electrode designs with arbitrary size and shape, and are arrayed only in a simple grid (e.g., a regular, repeating pattern of equally sized and shaped electrodes where each electrode is placed equidistant from its surrounding electrodes) with no consideration of the target anatomy or optimization for focal stimulation.

### Summary

Described herein is an improved multi-electrode pad with a plurality of electrodes (that are able to operate both independently and simultaneously) for transcutaneous stimulation. The electrodes can be designed in ways other than a regular repeating pattern of equally sized and shaped electrodes that are placed equidistant from one another. Additionally, the electrodes and/or the multi-electrode pad can be optimized based on any physiological feature, electrical property, and/or specific therapeutic end-use with each of the plurality of electrodes independently addressable through a single external cable. Additionally, systems and methods that can employ and/or manufacture the improved multi-electrode pad for transcutaneous stimulation are also described herein.

In one aspect, the present disclosure includes a multi-electrode pad for transcutaneous stimulation. The electrode pad can be configured to adhere to a patient's skin and provide transcutaneous stimulation to a portion of tissue. The electrode pad includes a flexible substrate. A plurality of electrodes can be arranged in an array on or within the flexible substrate. Each of the plurality of electrodes can be configured to apply a stimulation waveform. Conductive traces can be applied to the flexible substrate and coupled to each of the plurality of electrodes such that each of the plurality of electrodes is independently addressable through a single external cable.

In another aspect, the present disclosure includes a system that can employ a multi-electrode pad for transcutaneous stimulation. The system includes at least one flexible electrode pad, each comprising a plurality of electrodes arranged on or within a flexible substrate in an array and connected by conductive traces such that each of the plurality of electrodes is independently addressable through a single external cable. The system also includes a stimulator connected to the flexible electrode pad through the single external cable configured to provide a stimulation to at least a portion of the plurality of electrodes based on addresses associated with the at least the portion of the plurality of electrodes. A controller may be coupled to the stimulator comprising a processor configured to select the portion of the plurality of electrodes and to alter one or more parameters of the stimulation for the portion of the plurality of electrodes based on a user input.

In further aspect, the present disclosure includes a method for manufacturing a multi-electrode pad for transcutaneous stimulation. The method includes printing a first conductive layer, comprising a plurality of electrodes, traces, and connections, on a flexible substrate using conductive ink. The plurality of electrodes can be arranged in an array. A dielectric layer can be printed covering a portion of the conductive ink to insulate the traces, but leave connections and electrodes exposed. An adhesive layer can be placed on top of the dielectric layer. The substrate can be cut into a shape of an electrode pad.

### Brief Description of the Drawings

The foregoing and other features of the present disclosure will become apparent to those skilled in the art to which the present disclosure relates upon reading the following description with reference to the accompanying drawings, in which:
FIG. 1 is a block diagram of a system that can employ a multi-electrode pad for transcutaneous stimulation;
FIG. 2 is a schematic view of an example multi-electrode pad of the system of FIG. 1;
FIG. 3 is an example illustration of a multi-electrode pad (like that shown in FIG. 2) with a skeletonized substrate;
FIG. 4 includes example illustrations of materials that can be used for the electrodes of a multi-electrode pad (like that shown in FIG. 2) having variable sizes, patterns, and textures;
FIG. 5 is an example of different electrode geometries that can be used on a multi-electrode pad;
FIG. 6 is an example illustration of a multi-electrode pad (like that shown in FIG. 2) that includes additional components (in this case, light emitters for photobiomodulation);
FIG. 7 is an illustration showing example component layers that can make up a multi-electrode pad similar to the one shown in FIG. 2;
FIG. 8 is an example illustration of the system of FIG. 1 with the multi-electrode pad positioned at least partially on a neck of a patient;
FIG. 9 is a side view of a multi-electrode pad (like that shown in FIG. 2) held to a patient's skin when part of the system of claim 1;
FIG. 10 is an illustration of a multi-electrode pad (like that shown in FIG. 2) positioned on a patient and example orientation vectors of the multi-electrode pad as the patient changes positions;
FIG. 11 is an example illustration of how wireless communication may occur between a multi-electrode pad (like that shown in FIG. 2) and an external device;
FIG. 12 is an illustration of an example of multiple multi-electrode pads (each like that shown in FIG. 2) connected together via a hub;
FIG. 13 is an example with different views of another multi-electrode pad that is connected to an external connector and cable;
FIG. 14 is a process flow diagram illustrating a method for manufacturing a multi-electrode pad;
FIG. 15 is a process flow diagram illustrating a method for manufacturing a multi-electrode pad with a skeletonized substrate;
FIG. 16 is a process flow diagram illustrating another method for manufacturing a multi-electrode pad; and
FIG. 17 is a process flow diagram illustrating a method for treating a patient using a multi-electrode pad.

### Detailed Description

### I. Definitions

In the context of the present disclosure, the singular forms "a," "an" and "the" can also include the plural forms, unless the context clearly indicates otherwise.

The terms "comprises" and/or "comprising," as used herein, can specify the presence of stated features, steps, operations, elements, and/or components, but do not preclude the presence or addition of one or more other features, steps, operations, elements, components, and/or groups.

As used herein, the term "and/or" can include any and all combinations of one or more of the associated listed items. Use of the terms "and" and "or" alone should be read as "and/or" unless specifically mentioned that such an interpretation is not intended.

Additionally, although the terms "first," "second," etc. may be used herein to describe various elements, these elements should not be limited by these terms. These terms are only used to distinguish one element from another. Thus, a "first" element discussed below could also be termed a "second" element without departing from the teachings of the present disclosure. The sequence of operations (or acts/steps) is not limited to the order presented in the claims or figures unless specifically indicated otherwise.

When a feature or element is referred to as being "on" another feature or element, the feature or element can be directly on the other feature or element or intervening features and/or elements may also be present. However, when a feature or element is referred to as being "directly" on another feature or element, there are no intervening features or elements present. It will also be understood that, when a feature or element is referred to as being "connected", "attached" or "coupled" to another feature or element, it can be directly connected, attached, or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments. It will also be appreciated by those of skill in the art that references to a structure or feature that is disposed "adjacent" another feature may have portions that overlap or underlie the adjacent feature.

As used herein, and unless otherwise expressly specified, all numbers may be read as if prefaced by the word "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is +/- 0.1% of the stated value (or range of values), +/- 1% of the stated value (or range of values), +/- 2% of the stated value (or range of values), +/- 5% of the stated value (or range of values), +/- 10% of the stated value (or range of values), etc. Any numerical values given herein should also be understood to include about or approximately that value unless the context indicates otherwise. For example, if the value "10" is disclosed, then "about 10" is also disclosed. Any numerical range recited herein is intended to include all sub-ranges subsumed therein. It is also understood that when a value is disclosed that "less than or equal to" the value, "greater than or equal to the value" and possible ranges between values are also disclosed, as appropriately understood by the skilled artisan. For example, if the value "X" is disclosed the "less than or equal to X" as well as "greater than or equal to X" (e.g., where X is a numerical value) is also disclosed. It is also understood that the throughout the application, data is provided in a number of different formats, and that this data, represents endpoints and starting points, and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

As used herein, the term "transcutaneous stimulation" also referred to as "transcutaneous electrical nerve stimulation" or "TENS" can refer to the application of an electrical signal through the skin of a patient to stimulate a portion of tissue. For example, the portion of tissue can be at least a portion of a spinal cord, one or more spinal nerves, one or more peripheral nerves, or the like. One example use of transcutaneous stimulation is to provide pain relief via peripheral nerve, spinal nerve, and/or spinal cord stimulation. The transcutaneous stimulation can be delivered to the patient using one or more electrodes of a multi-electrode pad attached to the patient's skin in a certain position and orientation.

As used herein, the term "multi-electrode pad" (also referred to as an "electrode pad") can refer to a piece of flexible material that can include a substrate, at least one electrode, and conductive traces that can be attached to the skin of a patient (e.g., via an adhesive) so that the at least one electrode is in contact with the patient's skin.

As used herein, the term "substrate" can refer to a material that provides a surface on which conductive layers (e.g., conductive traces and electrodes), dielectric layers, and other electrical components can be deposited or inscribed. An example of a substrate is a flexible substrate that can bend without breaking and can be a thin, heat-resistant material that is typically made of polymers like polyimide and polyethylene terephthalate (PET). A substrate, such as a flexible substrate, can include one or more cuts to increase the bendability of the substrate. Another example of a substrate is a skeletonized substrate. The examples are not exclusive, for example a flexible substrate can include one or more cuts and can be skeletonized.

As used herein, the term "electrode" can refer to an electrical conductor within or on a substrate that can deliver an electrical stimulation. An electrode can include a specific interface between the body of the electrical conductor and the skin of the patient, such as but not limited to, a hydrogel interface. Two or more electrodes can be arranged in an array (e.g., a pattern of a plurality of electrodes) to provide a stimulating pattern of electric current or voltage.

As used herein, the term "conductive trace" can refer to a flat, narrow portion of conductive material that can conduct electricity to and from at least one component (e.g., from a connector to an electrode). A conductive trace is similar to a wire for conducting signals but uses substantially less space.

As used herein, the term "skeletonized" can refer to a substrate that is formed as peninsulas that surround only the locations where electrodes, traces, and contacts for connectors are printed, without the dead space of traditional substrates. Each electrode on a skeletonized substrate can move independently of other nearby electrodes on the skeletonized substrate if they are on separate peninsulas of substrate. A skeletonized substrate can allow an electrode array to conform to uneven skin or curved bodily surfaces more easily.

As used herein, the term "attachment" can refer to the action of attaching two things together, such as attaching one or more electrodes to the skin of a patient. Non-limiting examples of attachment mechanisms are an adhesive, a strap, tape, or the like. Attachment can be done removably or permanently.

As used herein, the term "patient" or "subject" can be used interchangeably and can refer to any warm-blooded organism including, but not limited to, a human being, a pig, a rat, a mouse, a dog, a cat, a goat, a sheep, a horse, a monkey, an ape, a rabbit, a cow, *etc*. The terms "patient" and "subject" can be used interchangeably herein.

### II. Overview

Transcutaneous stimulation applied to muscles and nerves has been used as a therapeutic and/or treatment for many disorders, injuries, and diseases. Examples of transcutaneous stimulation range from external defibrillation to activating muscles for strengthening or rehabilitation, modulating nerve signals to block pain or provide other functional improvement, facilitating the repair and regrowth of a damaged nerve, and/or up-regulating or down-regulating brain regions for applications including seizure prevention, tremor suppression, or treatment of conditions such as depression, stroke, traumatic brain injury, or the like. In each of these examples, the transcutaneous stimulation can be delivered by one or more skin surface electrodes. However, commercially marketed skin surface electrodes generally are not designed based on any physiological feature, electrical property, or specific therapeutic end-use, and generally have only a single active electrode site, with one cable required per electrode used to apply stimulation. Although electrode arrays with multiple electrodes on the same substrate have been developed, these arrays are impractical, unable to stimulate from all electrodes in the array independently and simultaneously, include electrode designs with arbitrary size and shape, and are arrayed only in a simple grid (e.g., a regular, repeating pattern of equally sized and shaped electrodes where each electrode is placed equidistant from its surrounding electrodes) with no consideration of the target anatomy or optimization for focal stimulation.

Described herein is an improved multi-electrode pad for transcutaneous stimulation and systems and methods that can employ and/or manufacture the improved multi-electrode pad for transcutaneous stimulation. This improved multi-electrode pad (or the electrodes therein/thereon) can be optimized based on any physiological feature, electrical property, and/or specific therapeutic end-use allowing for repeatable and consistent application of stimulation. The improved multi-electrode pad described herein can have a plurality of electrodes that are able to operate both independently and simultaneously and can be independently addressable through a single external cable. The improved multi-electrode pad can be designed with various electrode sizes, shapes, patterns, and materials rather than a traditional regular repeating grid pattern of equally sized and shaped electrodes. Connection to the electrodes can be achieved using a single connector and/or a single cable, thereby eliminating the hazards (e.g., mixed up cables, wires catching and pulling off the patient without being noticed, etc.) and difficulty of hooking up multiple cables (e.g., one for each electrode site). Additionally, sensors can be included with the electrodes on the multi-electrode pad so that sensing tasks and/or alternate therapeutic tasks can be performed using just the multi-electrode pad.

### III. Systems

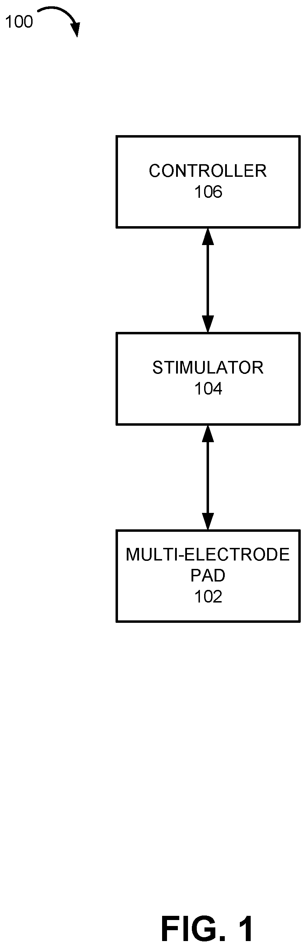

One aspect of the present disclosure can include a system 100 (FIG. 1) for transcutaneous stimulation. The transcutaneous stimulation can be provided for correcting heart function with external defibrillation, activating muscles for strengthening or rehabilitation, modulating nerve signals to block pain or provide other functional improvement, facilitating the repair and regrowth of a damaged nerve, up-regulating or down-regulating brain regions for applications, or the like. One particular example use for the system 100 is to provide transcutaneous stimulation to block pain; this use is particularly described herein, but this use is not meant to be the exclusive use of the system 100.

The system 100 can employ an improved multi-electrode pad 102 to apply the transcutaneous stimulation. The multi-electrode pad 102 can be flexible to accommodate different anatomical locations, shapes, and curvatures and can be further designed (e.g., with types and number of electrodes, shape/size of the pad, etc.) based on the specific anatomical part of a patient's body receiving a certain treatment. The multi-electrode pad 102 can include a plurality of electrodes that are able to operate both independently and simultaneously. The electrodes on the multi-electrode pad 102 can be a plurality of sizes and can be positioned in a variety of patterns. The electrodes and/or the multi-electrode pad 102 can be optimized based on any physiological feature, electrical property, and/or specific therapeutic end-use. Each of the plurality of electrodes on the multi-electrode pad 102 can be independently addressable through a single external cable. It should be noted that, although not illustrated in FIG. 1, the system 100 may include a plurality of multi-electrode pads, each with a different configuration and/or design for different uses and/or the same use.

The system 100 can also include at least a stimulator 104 connected to the multi-electrode pad through a single external cable and a controller 106 connected (wired and/or wirelessly) to the stimulator 104. It should be noted that although the controller 106 and the stimulator 104 are illustrated as different devices, the controller 106 may include at least some functionalities of the stimulator 104 or the stimulator 104 may include at least some functionalities of the controller 106.

The controller 106 can include at least a processor (e.g., any type of one or more electronic units designed to perform the functions of the processor). The controller 106 can have a memory coupled to the processor (e.g., the functionality may be implemented by separate chips). However, in some instances the memory and the processor can be implemented together (e.g., embodied within the same chip) (e.g., a microcontroller device). Optionally, the controller 106 can be in communication (wired or wireless) with an external device comprising at least one of a display (e.g., a video screen), another memory and/or another processor, and an input device (e.g., a keyboard, touch screen, and/or a mouse). As an example, the controller 106 and/or the stimulator 104 can be embodied as part of, or with, a computer, a tablet, or a set of buttons that when activated by a user (the subject or another user) can send stimulations to the one or more electrodes on the multi-electrode pad 102. The stimulation can include a pattern of activation of the one or more electrodes, determined by a preprogrammed setting or through a user interface.

The controller 106 can inform the stimulator 104 (a) that it is time to deliver a stimulation, (b) which one or more of the electrodes to deliver the stimulation through (according to the individual address/addresses of the electrode or electrodes), and (c) one or more parameters to set for the stimulation (different parameters can be set for the different stimulations through different electrodes). As an example, the controller 106 can store a record of the different addresses of the different electrodes in the multi-electrode pad. Based on a pre-programmed stimulation and/or a user input stimulation pattern (such as an on-the-fly stimulation selected by the patient or a caretaker of the patient), the controller 106 can select at least a portion of the plurality of electrodes of the multi-electrode pad 102 to deliver a transcutaneous stimulation. Additionally, the controller 106 can alter one or more parameters (e.g., amplitude, timing of repetitions, pulse width/shape, frequency, etc.) of the stimulation based on the pre-programmed stimulation and/or the user input stimulation pattern (e.g., the on-the-fly stimulation selected by the patient or a caretaker of the patient). In the case of an on-the-fly user input stimulation pattern, the controller 106 can ensure that safety boundaries are not exceeded according to one or more preset thresholds). For example, the controller 106 can limit the number of the parameters that may be altered at one time. The controller can limit the amount the parameter or parameters can be altered based on an algorithm intended to stimulate with a high focality and high intensity (e.g., the amount permitted for the adjustment is within a range permitted by the algorithm to ensure high focality and high intensity between the selected one or more electrodes).

The stimulator 104 can generate the stimulation according to instructions from the controller 106. The stimulator 104 can be connected to the multi-electrode pad 102 by a single, external cable. In some instances, a connector can couple the single, external cable to the multi-electrode pad 102. The connector can be external to the multi-electrode pad 102 or can be a part of the multi-electrode pad. However, a connector may not be needed and the electrode pad 102 can be coupled to the single, external cable in other known manners. In either case, the stimulator 104 can provide the stimulation to at least a portion of the plurality of electrodes of the multi-electrode pad 102 based on the addresses provided by the controller 106. Further, one or more ground electrodes can also be connected to and/or in electrical communication with the stimulator 104. For example, the stimulator 104 can be connected to the multi-electrode pad 102 by a single cable and connected to the one or more ground electrodes (e.g., placed at one or more different parts of the body away from the multi-electrode pad 102) by another one or more cables. Alternatively, at least one of the electrodes on the multi-electrode pad 102 can be selected as the ground electrode (and connected to the stimulator 104 with a different cable or the same cable).

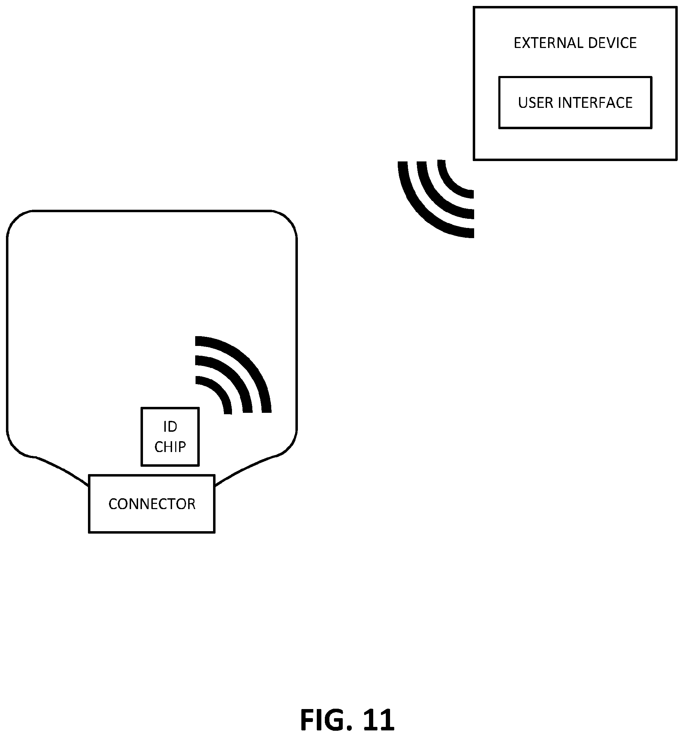

The multi-electrode pad 102 can receive the stimulus from the stimulator 104 through the single, external cable and provide the stimulus as a voltage signal or a current signal to the patient transcutaneously. The multi-electrode pad 102 can apply an electrical stimulation to a portion of a patient's tissue, including nerves (e.g., a portion of the spinal cord, one or more spinal nerves, one or more peripheral nerves, etc.) or muscles based on the stimulus. Each of the plurality of electrodes of the multi-electrode pad 102 can apply the same electrical stimulation or a different electrical stimulation. Additionally or alternatively, the multi-electrode pad 102 can perform other tasks in addition to applying electrical stimulation, including, but not limited to, measuring skin impedance at one or a range of frequencies, sensing analytes in the skin or sweat, delivering photobiomodulation, etc. Although a single multi-electrode pad 102 is illustrated in the system 100, it should be understood that the system 100 can include a plurality of multi-electrode pads 102. When a plurality of multi-electrode pads 102 are used, then each multi-electrode pad may be separately connected via a separate single, external cable to one or more stimulators 104 or each multi-electrode pad may be connected through the same single external cable (branched to connect to each multi-electrode pad, like through a hub (described in further detail in FIG. 11) - which may be flexible) and the same stimulator. Each of the at least one multi-electrode pad can include a plurality of electrodes arranged on or within a flexible substrate in an array. The plurality of electrodes on a single multi-electrode pad can be connected by conductive traces such that each of the plurality of electrodes is independently addressable through a single external cable.

The multi-electrode pad 102 can be attached a patient's skin and provide transcutaneous stimulation to a portion of the patient's tissue (e.g., peripheral nerve, spinal cord, spinal nerve, muscle, or the like). For example, the multi-electrode pad 102 can be attached to the patient's skin with an adhesive so that the multi-electrode pad 102 can adhere to the patient's skin such that contact between the one or more electrodes on the multi-electrode pad can be maintained during patient movement or skin deformation without the need for tape or straps. A multi-electrode pad 102 for a given anatomical location may come in one size, two sizes, more than two sizes, etc., to accommodate patients of different sizes to provide optimal therapy and improved performance to patients of different body types. For example, a "standard" size may be appropriate for most adults, while a smaller size may be appropriate for children or small adults. Sizing differences and electrode pattern differences between each multi-electrode pad 102 may be based on simple scaling (e.g., by scaling sizes based on volume ratio or height ratio between different demographics). Alternately, size and electrode pattern differences may be based on simulation results using anatomical models of different people or groups of people.

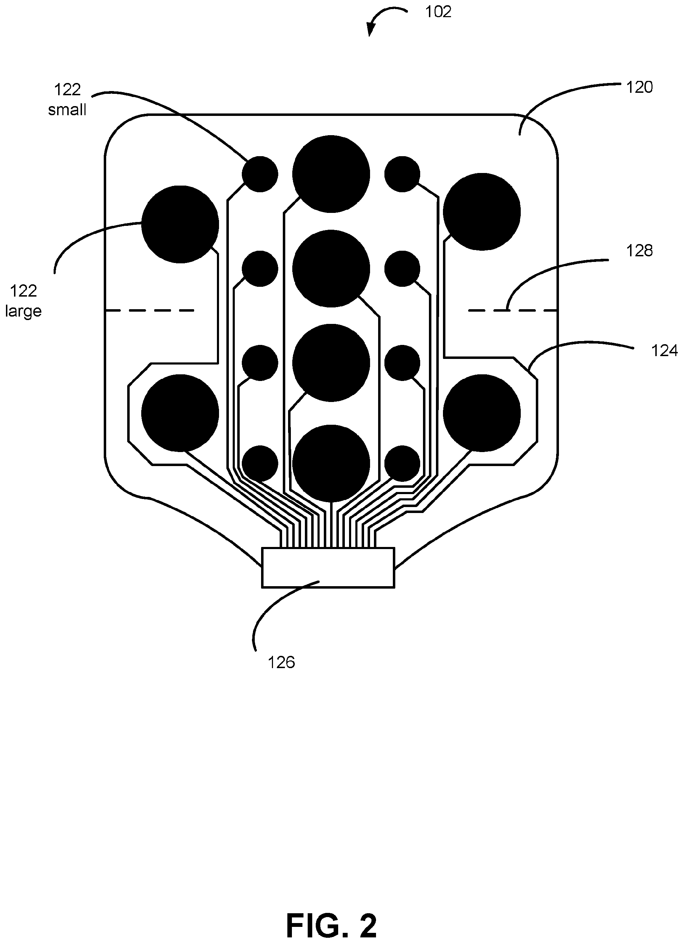

An example, multi-electrode pad is shown in FIG. 2. The multi-electrode pad 102 can be formed using a substrate 120. The substrate 120 is shown with a predominantly rounded rectangular shape but can be shaped in any shape that can be attachable to a target portion of a patient's body (e.g., by an adhesive layer) and can facilitate the therapy to be delivered. The substrate 120 can be a flexible substrate (which can be bent, folded, creased, etc.). A flexible substrate is preferred to a rigid substrate because the flexible substrate can conform to a patient's body and maintain a position during movement without causing the patient additional discomfort. The multi-electrode pad 102 may or may not include an extra foam layer between the substrate and a skin adhesive; the addition of a foam layer would add additional comfort during long-term use, while a design with no foam layer would be thinner and more flexible.

Generally, a flexible substrate is a thin material, which is often heat-resistant, made of one or more polymers of other suitable material. For example, the substrate material can include thermoplastic polyurethane (TPU), polyethylene terephthalate (PET), polyimide, silicone, Parylene, or the like. The substrate 120 can include one or more slits or cutouts (shown as cuts 128 in FIG. 2) that can help the substrate 120 to conform to irregular or curved areas of a patient's body. As shown in FIG. 2, the cuts 128 can be two slits placed approximately halfway up the multi-electrode pad 102 and on either side of the multi-electrode pad to conform to the curve of the neck, where changing in size as the neck transitions into the upper back necessitates different radiuses of curvature for the top and bottom sections of the multi-electrode pad 102. Other cuts 128 of any given length and position through the substrate 120 can be included in areas that do not include electrical components to allow the electrode pad to conform to additional anatomical areas of non-uniform curvature.

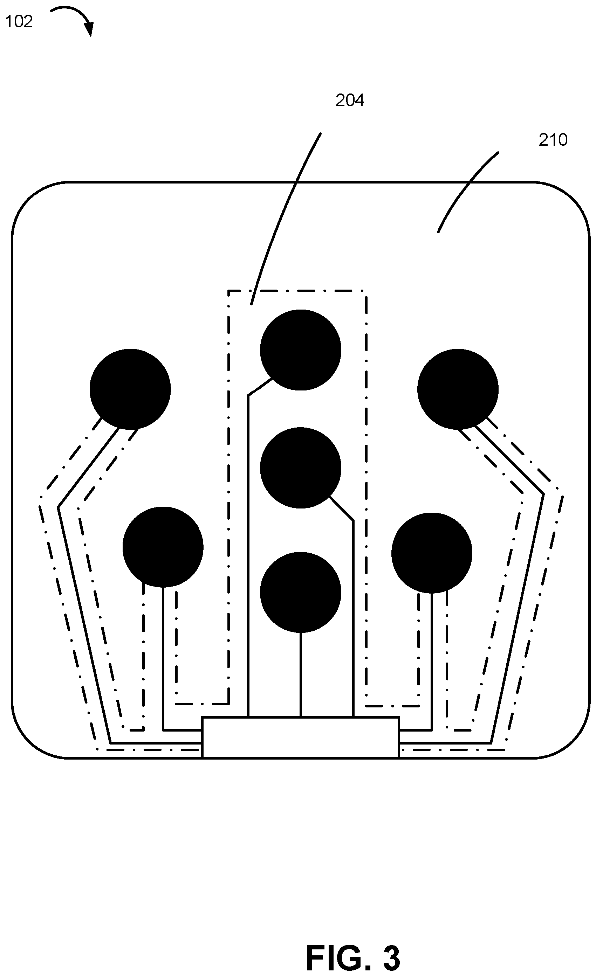

Alternatively, as shown in FIG. 3, the substrate 120 can be skeletonized - with a stretchable fabric or polymer layer as a backing layer 210, with electrodes and traces integrated onto a skeletonized layer 204 (e.g., formed of TPU, PET, or another flexible but less stretchable substrate). Skeletonizing the design can involve placing cuts in the substrate such that individual electrodes or groups of electrodes would be placed on "peninsulas" separated from adjacent groups except for at a common mating point (i.e. near the connector); this construction would allow each electrode in the pad to move independently with the skin while maintaining orientation and ensuring the integrity of electrical connections, solving a major issue that prevents the adoption of electrode arrays with non-stretchable backing from being used during normal daily activities.

Referring again to FIG. 2, the multi-electrode pad 102 includes a plurality of electrodes 122 (large and small), each configured to apply/deliver a stimulation. The plurality of electrodes 122 can be arranged in an array on or within the substrate 120. One or more of the plurality of electrodes 122 in the array can be positioned to focus an electric field on one or more areas of tissue based on the pattern of stimulation applied therethrough (e.g., to stimulate a specific portion of tissue such as a spinal nerve, ganglion, spinal segment, or the like). The plurality of electrodes 122 may include electrodes of multiple sizes. For example, in FIG. 2, illustrated are eight large electrodes (e.g., 2 cm diameter) located at the left side, the right side, and the center of the multi-electrode pad 102 and eight small electrodes (e.g., 1 cm diameter) located on the left side of the middle and the right side of the middle of the multi-electrode pad (such that there are sixteen independently addressable circular electrodes of two different sizes). While sixteen electrodes of two different sizes and the configuration described above are shown in FIG. 2, there can be greater or fewer number of sizes of electrodes and different numbers of electrodes of different sizes, shapes, etc. in different configurations (limited by manufacturing capabilities). As an example, the positions and sizes of the plurality of electrodes (as well as parameters for a stimulation determined by the controller 106) can be determined based on anatomical models generated based on one or more images (e.g., MRI images, CT images, or the like) and tested in simulation (using an optimization algorithm, such as the optimization algorithm described in detail in WO2018106843 A1, which is incorporated by reference in its entirety).

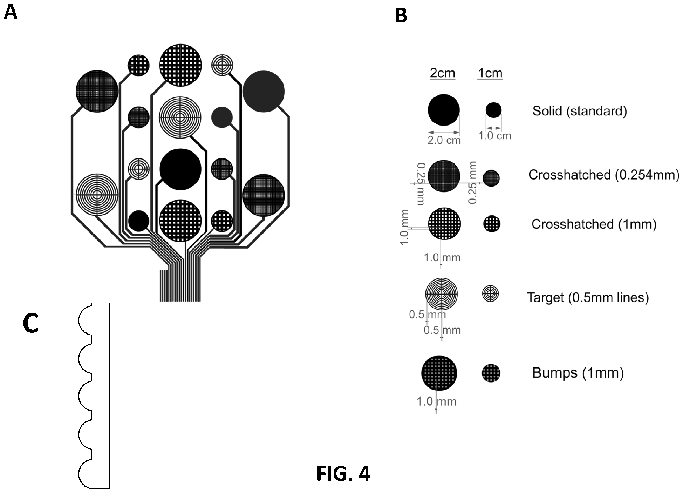

The plurality of electrodes 122 can be composed of a same material and same flat surface (as shown in FIG. 2) or the plurality of electrodes 122 of one or more different materials or different surfaces (e.g., FIG. 4). It should be noted that at least one additional interfacing layer (e.g., a hydrogel layer, a layer of another material like a carbon nanotube-filled epoxy material, etc.) can be placed between the plurality of electrodes 122 and the skin of the patient. Additionally, an adhesive layer can cover at least a portion of the substrate 120 and the conductive traces without covering the plurality of electrodes 122 to isolate the electrical sites and provide stable adhesion to the skin.

In addition to the plurality of electrodes of the multi-electrode pad being able to be different sizes (may be dynamically adjustable) and/or shapes, the electrodes can be different materials and/or have different surface textures. As shown in FIG. 4, printed patterns may be added to the skin facing side of one or more of the plurality of electrodes to reduce the edge effect, lower impedance, and/or allow higher amplitude stimulation without causing discomfort. These patterns may include crosshatches or target-like patterns; in addition, a second printed conductive layer may be used to add "bumps" or raised patterns in a direction normal to the surface of the electrodes (e.g., a hemispherical bump) to increase electrode surface area in contact with the patient. As shown in FIG. 4, elements A and B show examples of different surface patterns that can be used, and element C shows an example texture of the material of one or more of the electrodes having protruding bumps rather than being flat. At least one of the plurality of electrodes can be designed as shown in FIG. 4. The patterns can be chosen based on a type of tissue being stimulated. For example, at least one of the plurality of electrodes can include a surface with a pattern designed to spread electric field evenly or to reduce impedance between the at least one of the plurality of electrodes and the tissue being contacted by the at least one of the plurality of electrodes . Additionally, while the electrodes are shown as circles in FIG. 4 and throughout the electrodes can be any different shapes such as an ellipse, an oval, an annulus, or any other geometric, non-geometric, or non-symmetric shape.

The purpose of these different shapes may be to more finely control the electric field generated in the body, to allow more efficient implementation of an optimization algorithm, to differentiate electrodes based on their preferred functions (e.g., if one electrode is often configured as ground it may be larger or shaped so as to have a uniform distance from other electrodes), or to lower impedance.

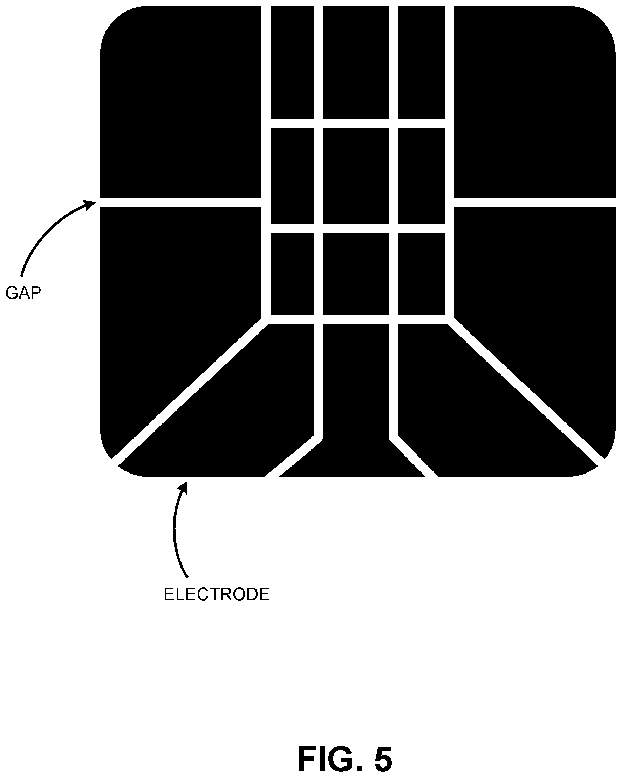

FIG. 5 shows an example of alternative electrode shapes / geometries that can be used by the multi-electrode pad. In this example, large electrodes can be used to minimize impedance. For lowering impedance, in general a larger electrode area is preferred, so one example of a non-symmetric electrode shape may involve placing the centroid of each electrode in an area prescribed based on the target anatomy for stimulation and then increasing the area until all electrodes are only separated by a thin border of non-conductive material.

Additionally or alternatively, one or more of the plurality of electrodes can be functionalized to detect a chemical and/or biological signature of a condition of the patient's body, such as inflammation of tissue in contact with one or more of the plurality of electrodes, allergic reaction of tissue in contact with one or more of the plurality of electrodes, biomarkers in sweat, impedance of skin under one or more of the plurality of electrodes, or the like. For example, one or more of the plurality of electrodes can detect an impedance between 100-1000 Hz to detect inflammatory biomarkers, such as IL-18 or C-reactive protein (CRP) with the addition of a thiol cross-linker functionalized with the biomarker-specific captured antibody to an electrode's surface. This could be useful to alert the patient if the pad was being worn for too long or if there was an allergic reaction occurring. In addition, other therapies could be combined with stimulation therapy on the pad.

Referring again to FIG. 2, the plurality of electrodes 122 can be laid on and/or embedded within a substrate 120. Each of the plurality of electrodes 122 can be associated with a unique conductive trace 124. With each of the plurality of electrodes 122 associated with its own unique conductive trace 124, each of the plurality of electrodes 122 can operate individually. However, virtual electrodes can be formed from two or more of the plurality of electrodes with synchronized stimulation timings. Such a virtual electrode may be able to change size and shape dynamically based on how many electrodes in the configuration are used.

Conductive traces 124 may be printed on the same side of the substrate as the plurality of electrodes 122 contact the skin, on the opposite side of the substrate as the plurality of electrodes 122, or both; traces may make electrical connection between the top and bottom sides of the trace through vias or holes filled or coated with conductive material. In some cases, multiple layers of conductive traces 124 may be created by layering multiple substrates 120. A plurality of conductive traces 124 may be deposited on the substrate 120. The plurality of conductive traces can be, for example, insulated silver traces or copper traces. The plurality of conductive traces 124 can be applied to the substrate 120 and coupled to each of the plurality of electrodes 120 such that each of the plurality of electrodes 122 is independently addressable through the single external cable to provide independent stimulation by each of the plurality of electrodes 122. The connector 126 can couple the plurality of electrodes 122 to the single cable via the ends of the conductive traces 124. However, it should be understood that the connector 126 may be integrated with the multi-electrode pad 102 and/or outside (external) to the multi-electrode pad 102 (e.g., not integrated with the multi-electrode pad during manufacture). The connector 126 can be placed on the opposite side of the substrate 120 as the active electrode sites to avoid skin contact; this may be achieved using electrical vias in the substrate 120 or by bending the substrate 120. The connector 126 can be a push-pull connector or other mechanical connector, or it may be replaced with a magnetic connector that can clip on and off easily while maintaining a strong connection during therapy. In one example, the magnetic connector can sit at the center of the multi-electrode pad 102 on an opposing side to the plurality of electrodes 122.

There may be additional conductive traces 124 extending from the connector 126 (not shown in FIG. 2) that are intended for additional elements like sensors (e.g., one or more of an inertial measurement unit that may include one or more components to measure an orientation angle of the electrode array and/or the multi-electrode pad 102, a linear acceleration of the electrode array and/or the multi-electrode pad, and/or a radial acceleration of the electrode array and/or the multi-electrode pad; a bend sensor that includes one or more components that measure a bending moment of the electrode array and/or the multi-electrode pad; an electromyogram (EMG) sensor; a near-infrared spectrum (NIRS) sensor; or the like), photobiomodulation units, and/or additional devices, etc. The sensors, photobiomodulation units, additional devices, or the like can communicate with the stimulator 104 and/or the controller 106 through either additional pins in the connector 126 or wirelessly. In another example, a temperature sensor could be integrated into the multi-electrode pad to provide information on patient metabolic activity or stress levels, to detect fever, or to detect if there is any inflammation below the stimulation site. If the devices in the multi-electrode pad 102 communicate wirelessly, power could be provided by additional pins in the connector 126.

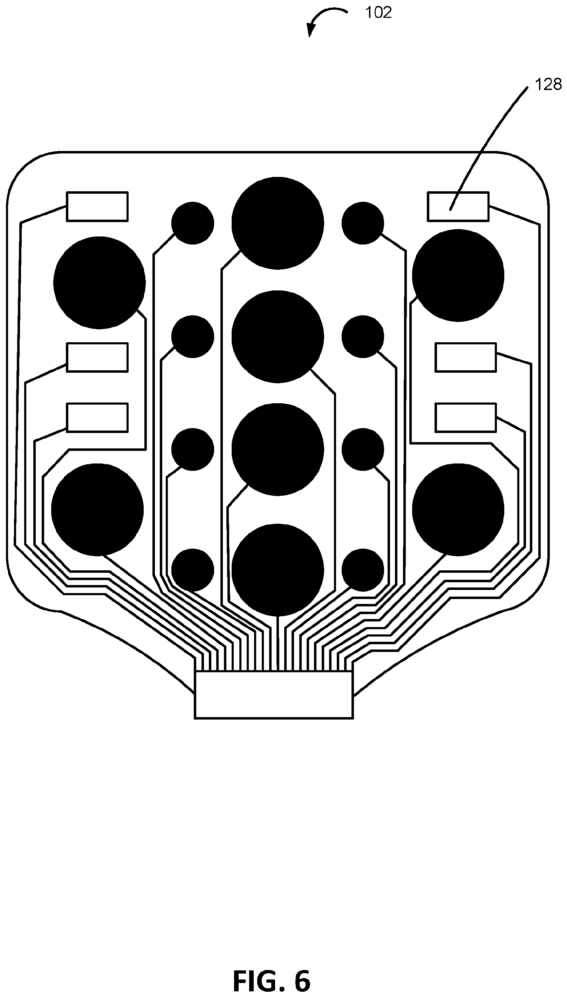

For example, in FIG. 6, additional conductive traces 124 go to a plurality of light sources (also referred to as light emitters) 128 (part of photobiomodulation units) arranged on or in the substrate 120. Each of the plurality of light sources 128 can provide photobiomodulation therapy to surrounding tissue. Six light sources 128 are shown, but any number needed for photobiomodulation therapy is contemplated. However, it should be noted that the additional elements may also be located external from the multi-electrode pad 102. Photobiomodulation therapy and electrical stimulation can be combined by an electrode pad 102 including light sources (LEDs, laser diodes, or other devices) integrated alongside the plurality of electrodes (122 of FIG. 2) and connected to the same substrate (120 of FIG. 2) and printed conductive traces (124 of FIG. 2), as shown in FIG. 6. The same or different but simultaneous stimulation signals used for electrical neuromodulation can be used to activate the light emitters, allowing light therapy and stimulation therapy to proceed in parallel. Photobiomodulation therapy may be used for a number of medical applications, including providing a second stimulation modality to modulate nerves underneath or around the pad (with potentially synergistic effects when combined with electrical stimulation), accelerating wound healing or healing of an injury such as spinal cord injury, or reducing inflammation / reaction to stimulation in the skin or proximal tissue. Similarly, ultrasound transducers (not shown) may be integrated alongside electrodes and connected to the same substrate and traces. Ultrasound may be used to provide an additional therapeutic modality through neural activation or to monitor muscle/tissue movement during therapy to aid in therapy calibration or closed loop stimulation. Another example can involve placing a resistance on the multi-electrode pad 120 using a surface mount resistor device, a serpentine trace, or another method of generating a known resistance or impedance; this known resistance could be used to calibrate impedance measurement in the stimulation device.

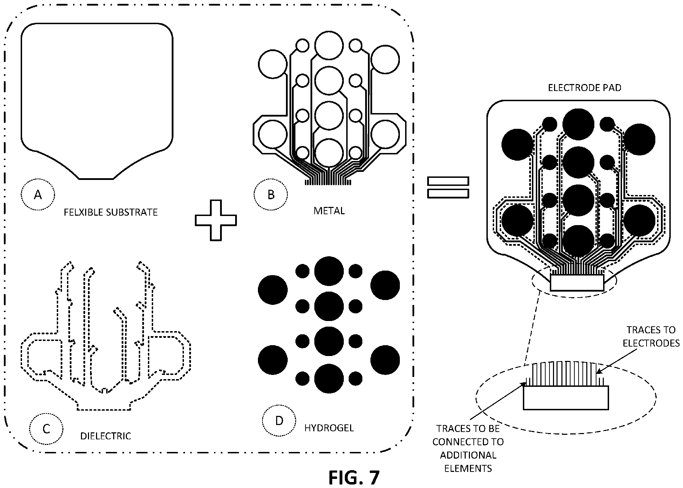

An example of a way the multi-electrode pad 102 can be assembled is shown in FIG. 7. A flexible substrate is acquired and or cut into a specific electrode pad shape at A. Then one or more conductive metal inks can be screen printed onto the substrate at B. The printed conductive metal inks can define the electrode sites, conductive traces, and connections (for the connector, which are ends of the conductive traces) within the multi-electrode pad. At C, a dielectric layer can be screen printed on top of the conductive traces s to insulate the conductive traces from the body, but the electrode sites and the connections can be left uncovered. Additionally, not shown, an adhesive layer can be positioned to cover at least a portion of the substrate and the dielectric layer, but not covering the electrode sites or the connections. The adhesive layer can be positioned such that it will contact a portion of a patient's skin. The adhesive layer can also include a backing that can be peeled off for use. Also not shown, a connector may be applied to the substrate using conductive epoxy or low-temperature solder such that the connections are in electrical communication with parts of the connector. At D, hydrogel, for a hydrogel interface, can be applied only on top of the active electrode sites; this hydrogel could be dispersed as a liquid and UV cured or could be cut from a sheet and then applied. The electrode pad can be cut into its shape using die cutting, CNC cutting, or laser cutting (this can occur before or after any step in this process). A foam layer may be placed between the dielectric and the skin adhesive to add comfort to the patient. The order of steps and the need for all the steps or additional steps may be varied based on convenience, time, ease of manufacturing, or the incorporation of different features into the multi-electrode pad.

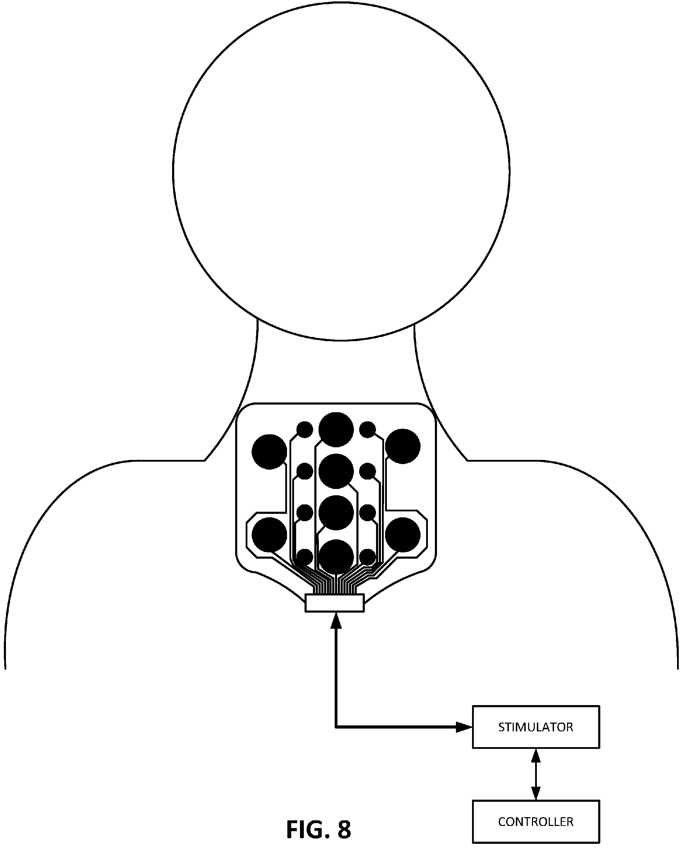

The system 100 (with a multi-electrode pad, a stimulator, and a controller) can be used, as an example, to deliver a stimulation to a patient's neck, as shown in FIG. 8. In this example, one multi-electrode pad may be placed on the skin above the cervical spinal cord to provide stimulation to nerves innervating the upper body. In some instances, a second multi-electrode pad may be applied on the skin above the lumbar spinal cord to provide stimulation to nerves that innervate the lower body. The electrode pad or pads may also be used with one or more ground electrodes placed on the skin or use one or more electrodes in the array as ground electrodes. Although not illustrated, the multi-electrode pad may contain slits on each side to help conform to the neck and maintain adhesion during neck movement.

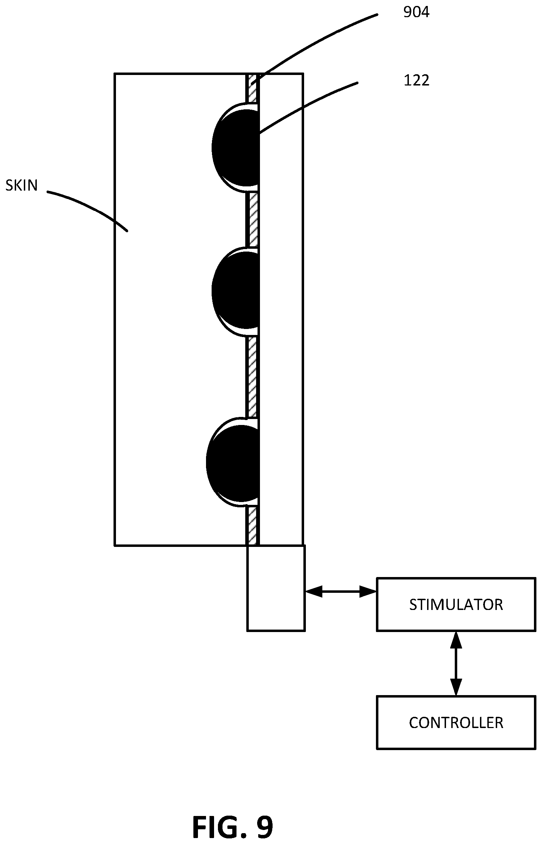

As shown in FIG. 9, the plurality of electrodes 122 can be formed in a three-dimensional rounded shape so that it maintains contact with the skin or other deformable surfaces at all times and the adhesive 904 can be applied to areas other than the electrodes.

Using a setup similar to FIG. 8 (or the same as FIG. 8), spinal nerves can be stimulated to restore movement after spinal cord injury; for this purpose, the multi-electrode pad can be placed over the spinal cord near (above, below, or on top of) the site of injury and the stimulation pulses would be configured to activate nerves needed for movement or excite the spinal cord network. In one example, different nerves could be activated as a patient transitioned from moving their hand forward, grasping an object, and pulling it back. The nerves controlling the muscle groups for these three movements are anatomically close together and a traditional electrode could not provide focused stimulation to activate the nerves at different times; an advantage of the electrode pad discussed here is that it could for the first time enable precise transcutaneous stimulation for achieving therapy in this way.

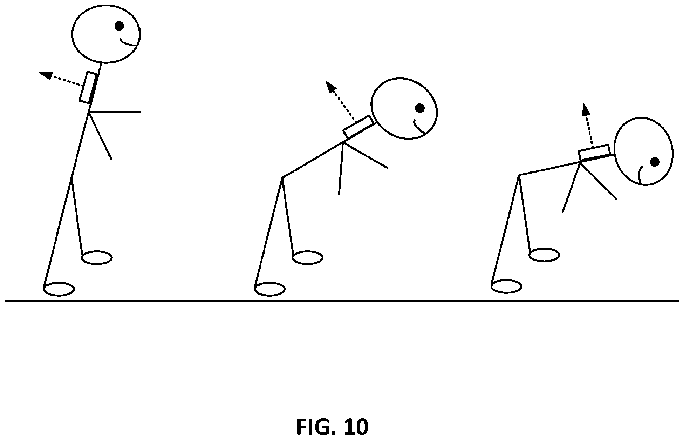

Additional components of the system can include sensors placed on the patient's body (on the multi-electrode pad or external thereto) to help calibrate and control stimulation during movements. For example, one or more inertial measurement units (IMUs) could be included to measure the position and orientation of the patient or the curvature of the multi-electrode pad 102, which would be useful to vary stimulation amplitude or other parameters to achieve maximum treatment efficacy without patient discomfort. FIG. 10 shows the use of a position sensor integrated within the multi-electrode pad to report an orientation vector (represented as dashed arrows) in real time during therapy, allowing for closed loop titration of the stimulation signal strength or variation of the therapy based on the patient's activity. In the above example, sensors could be placed on the muscles of the arm and shoulder to detect arm position (for example, using inertial measurement units) and/or muscle activation (for example, using electromyogram (EMG) or near-infrared spectrum (NIRS)-based sensors). The sensors can detect when the arm is in the proper position and send signals to a controller so that controller can transition the stimulation pattern from "reaching" to "grasping" etc. or the sensors in combination with the controller can detect intent through subthreshold EMG measurements and increase stimulation amplitude until muscle activation is detected through movement and NIRS detection. The system can also encompass automatically titrating stimulation up or down depending on the position of the patient's body or the patient's spinal curvature; transitioning through stimulation of various nerves to replicate stepping; stimulating spinal nerves that enervate trunk muscles to control posture; or any other application involving varying stimulation focus point with movement, position, or patient intent. Importantly, the addition of an IMU or other sensor in the multi-electrode pad disclosed here would allow the titration or variation of stimulation with body position, spinal curvature, or movement without requiring additional sensor electronics.

The system can also be used to improve autonomic function in patients with impaired autonomic function, which is often a side effect of spinal cord injury. Autonomic functions include bowel function, bladder function, blood pressure, heart rate, heart rate variability (HRV), lung function, and immune system function. The system can be used to treat any of these functions, alone or in combination with sensors (such as heart rate or blood pressure sensors) to provide feedback. In addition to spinal cord injury, the system can be used in a similar manner to treat symptoms of cerebral palsy, Parkinson's disease, essential tremor, stroke, amyotrophic lateral sclerosis (ALS), or another disease that involves impaired neural communication between the brain and body. The system can also be used to treat pain through selectively modulating nerves that transmit pain signals to the brain.

Additional features built into the multi-electrode pad 102 could include features for calibration or verification of genuine multi-electrode pads (as compared to non-authorized, non-compatible, or "counterfeit" electrode pads). For example, a cryptographic integrated circuit (IC) can be included in the pad and connected to additional pins in the connector using more printed traces; this IC could be used to verify that the pad was genuine before providing stimulation, to alert the user if the pad was expired or had been previously used, or to store calibration information or information about the electrode sizes and positions on this multi-electrode pad 102. As shown in FIG. 11, a cryptographic identification chip (ID chip) can be integrated into the multi-electrode pad and may communicate with a user interface of an external device (e.g., the controller or other computing device) wirelessly or through the same cable used to provide stimulation (wireless communication is shown in FIG. 11).

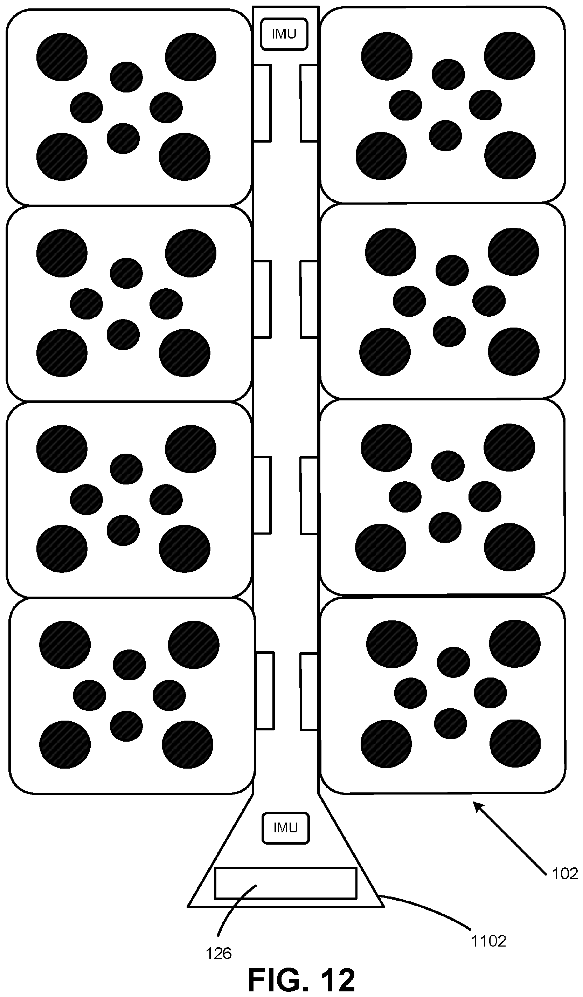

As briefly described above, multiple multi-electrode pads can be used with a connecting hub structure. FIG. 12 shows an example of multiple multi-electrode pads 102 (eight are shown but any number greater than or equal to two is contemplated) connected together via an exemplary flexible hub structure 1102. The flexible hub 1102 can be any shape conducive to positioning a plurality of multi-electrode pads 102 around it. The flexible hub 1102 can include the single external cable that connects at single cable attachment site 126 that can route electrical stimulation to one or more an appropriate electrodes or multi-electrode pads 102. The flexible hub 1102 can include a plurality of connectors that can allow connection to each of the plurality of multi-electrode pads. Inside the flexible hub 1102, electrical traces or wires can rout stimulation signals from the single cable attached at single cable attachment site 126 to each electrode in the array via the connections to the multi-electrode pads. In one version of the flexible hub 1102, a multiplexing circuit can be contained inside the hub to allow selection of which pad to stimulate using signals from the cable. A multiplexing circuit can allow the cable to be lightweight and flexible while still providing stimulation to multiple electrodes on multiple multi-electrode pads 102. The flexible hub 1102 may also contain some sensors (shown as IMUs, but may also be EMG sensors, temperature sensors, curvature sensors, or the like). In an example, the flexible hub 1102 may include a flexible circuit board overmolded with silicone rubber, with connectors on each side for multi-electrode pads 102, a cable attachment connector at the bottom for the single external cable, and adhesive on one side to attach the hub to the skin. The cable can send stimulation pulses to any electrodes connected to the hub 1102 and can return measurement signals from sensors embedded in the hub 1102.

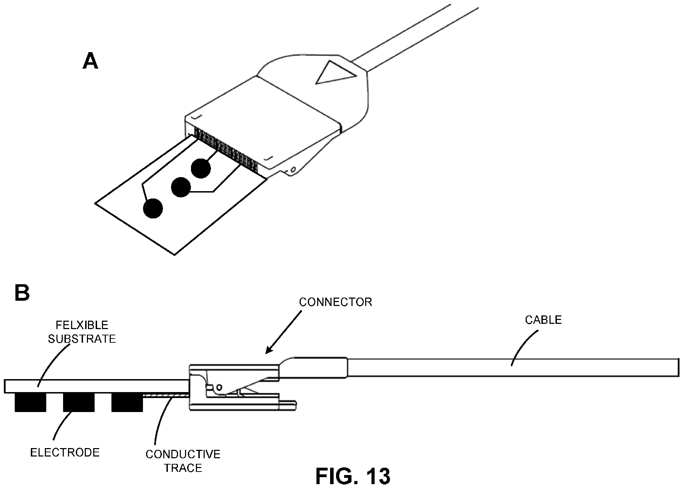

Another example of a multi-electrode pad having no connector on the pad itself is shown in FIG. 13. In this example the electrodes can be attached to conductive traces that terminate at exposed connector connections at one edge of the electrode pad. To achieve electrical connection to a stimulation or recording electronics systems (e.g., a stimulator 104 and controller 106 in FIG. 1), an external connector attached to a cable can be used. The exposed connector connections on the electrode pad can each contact a conductive pin within the external connector. For example, an external connector may be formed like a clamp with a first portion and a second portion that hinge around a pin and can be open and shut by applying pressure to a spring hinge. The external connector can include at least one or more components that can receive and connect with the exposed connector connections on the electrode pad in the first and/or second portions. This embodiment would be advantageous in that no horizontal force would be placed on the exposed pads during connection, reducing the risk of damage to the contacts.

Another example connector (not shown) can include a mechanical snap feature that could be moved to an "open" position to allow insertion of the contact pads with zero force and a "closed" position that would hold the contact pads in place through friction. A third design (not shown) can involve spring-loaded contacts in the connector where the contact pads on the electrode pad would slide into the connector and make electrical contact through the spring-loaded pins. For any of these examples, mechanical features may be used to align the contact pads to the pins; one example of an alignment feature may be the use of one or multiple non-conductive bumps or columns that aligns with a hole on the electrode pad. The contact pads on the electrode pad may be printed on the same substrate using the same conductive materials as the electrodes themselves; doing so would simplify manufacturing. Alternately, a stiffener or thicker material may be placed on the substrate opposite the contact pads to increase robustness when inserted into the connector.

### IV. Methods

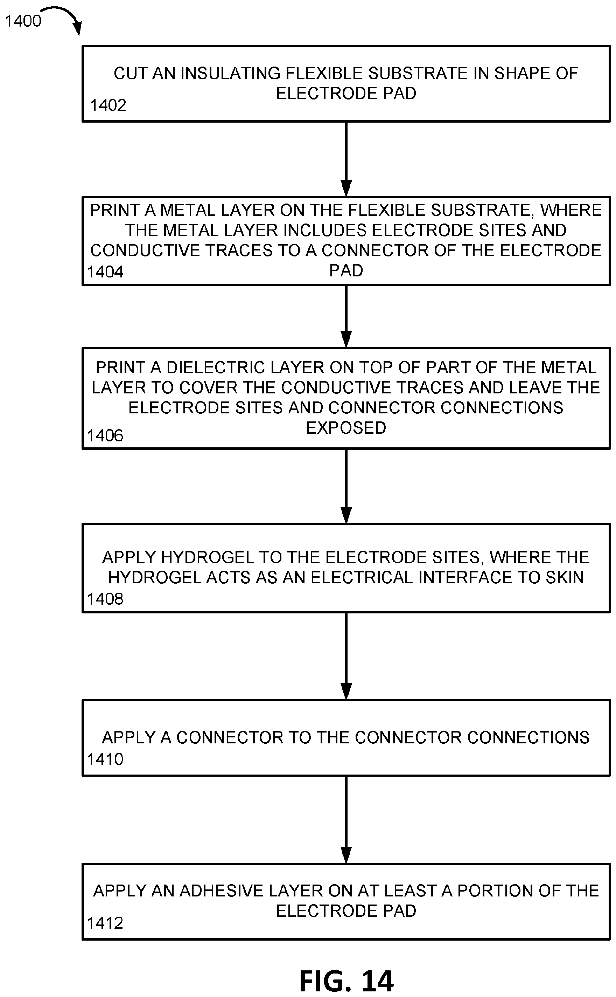

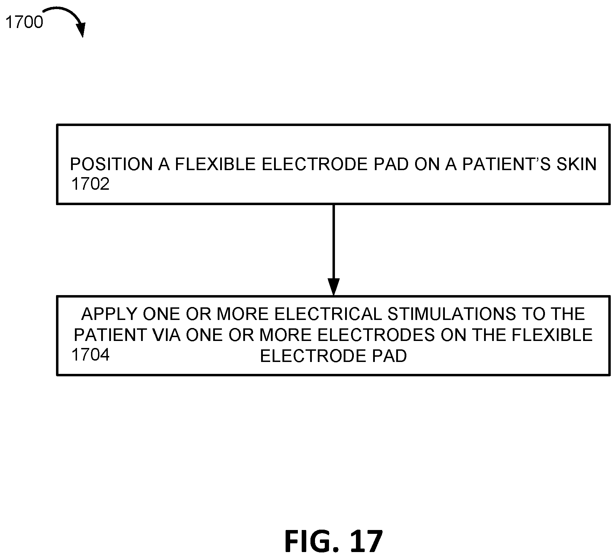

Another aspect of the present disclosure can include methods 1400-1600 for manufacturing the improved multi-electrode pad (FIGS. 14-16), as well as method 1700 for employing the improved multi-electrode pad (FIG. 17). The multi-electrode pad can be any one or more of the multi-electrode pads shown in FIGS. 2-13. Additionally, the multi-electrode pad can operate in the system of FIG. 1. For purposes of simplicity, the methods 1400-1700 are shown and described as being executed serially. However, it is to be understood and appreciated that the present disclosure is not limited by the illustrated order as some steps could occur in different orders and/or concurrently with other steps shown and described herein. Moreover, not all illustrated aspects may be required to implement the methods 1400-1700, nor are methods 1400-1700 limited to the illustrated aspects.

In the methods 1400-1600 of FIGS. 14-16, a multi-electrode pad (e.g., multi-electrode pad 102) can be manufactured. A first conductive layer, comprising a plurality of electrodes, traces, and connections, can be positioned (e.g., printed, etched, etc.) on a flexible substrate using conductive ink. A dielectric layer can be printed over at least a a portion of the conductive ink to insulate the traces but can leave the connections and electrodes exposed. An adhesive layer can be placed on top of the dielectric layer. The substrate can be cut into a shape of an electrode pad. The plurality of electrodes printed on the multi-electrode pad can be arranged in an array. Each of methods 1400-1600 describe variations of this method.

FIG. 14 illustrates a method 1400 for manufacturing a multi-electrode pad. At 1402, an insulating flexible substrate can be cut into a shape of an electrode pad. At 1404, a metal layer (or alternatively a conductive layer composed of a non-metal material) can be printed on the flexible substrate, where the metal layer includes electrode sites and conductive traces to a connector of the electrode pad. Additionally, a second conductive layer can be printed on top of the metal layer to form three-dimensional patterns on at least one of the plurality of electrodes. At 1406, a dielectric layer can be printed on top of part of the metal layer to cover the conductive traces and leave the electrode sites and connector connections exposed. At 1408, a hydrogel (e.g., an isolated hydrogel section) can be applied to (placed on top of) the electrode sites, where the hydrogel acts as an electrical interface to skin. Other known types of interfaces can alternatively be applied to the electrode sites in place of a hydrogel. At 1410, a connector can be applied to the exposed parts of the traces that act as the connector connections. Electrical connections can be made between the connector or elements connected with the connector and the traces on the electrode pad. At 1412, an adhesive layer can be applied on at least a portion of the electrode pad. A foam layer can be placed between at least a part of the substrate and the adhesive layer.

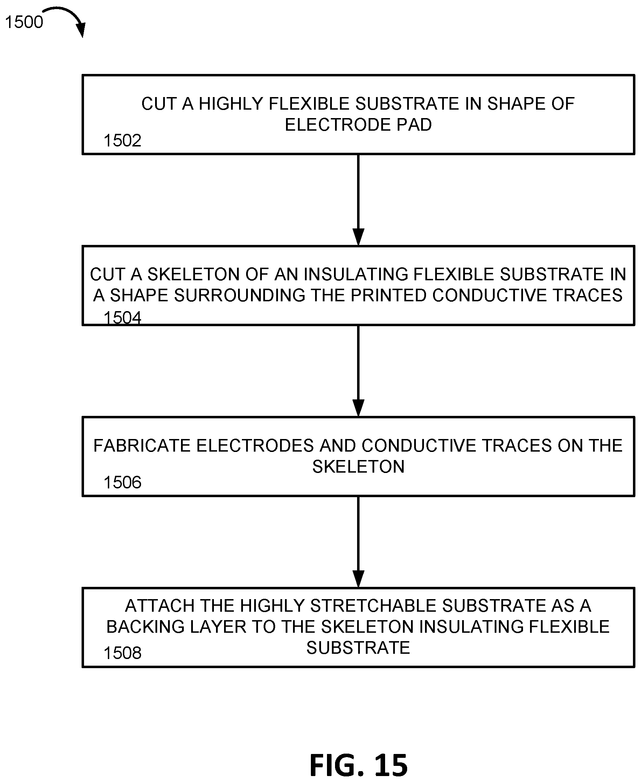

FIG. 15 illustrates a method 1500 for manufacturing a multi-electrode pad with a skeletonized substrate. At 1502, a highly flexible substrate can be cut in the shape of an electrode pad to act as a backing layer. At 1504, a skeleton of an insulating flexible substrate can be cut in a shape surrounding the locations for printed conductive traces and electrodes. Such a skeleton can include a plurality of peninsula-like protrusions. At 1506, electrodes and conductive traces can be fabricated on the skeleton by any known method. At 1508, the highly stretchable substrate can be attached as a backing layer to the skeleton insulating the flexible substrate. The skeletonized pattern allows one or more electrodes in an array to move independently from other electrodes on a separate peninsula of insulating flexible substrate.

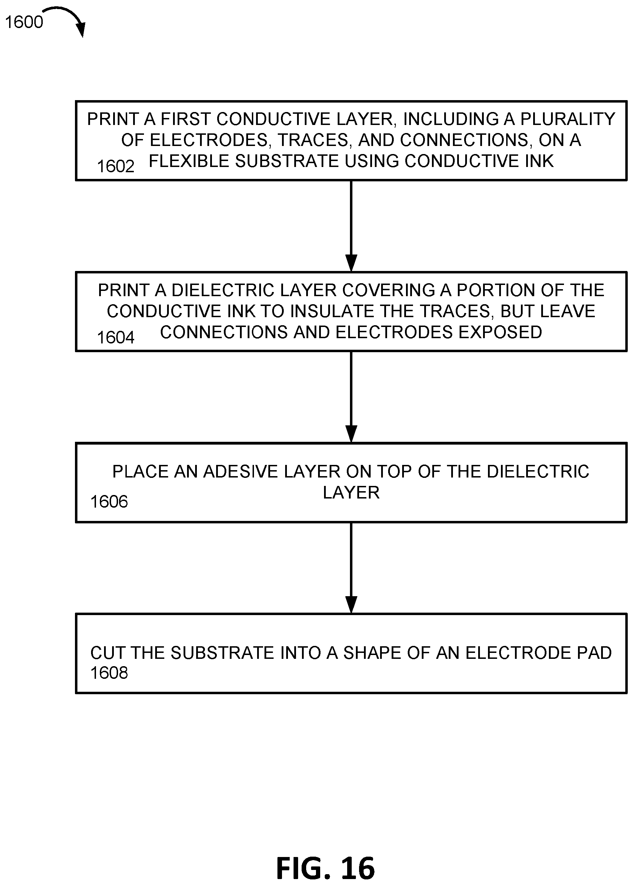

FIG. 16 illustrates another method, method 1600, for manufacturing a multi-electrode pad. At 1602, a first conductive layer, including a plurality of electrodes, traces, and connections, can be printed on a flexible substrate using conductive ink. At 1604, a dielectric layer can be printed covering a portion of the conductive ink to insulate the traces while leaving the connections and electrodes exposed. At 1606, an adhesive layer can be placed on top of the dielectric layer. At 1608, the substrate can be cut into the shape of an electrode pad.

FIG. 17 illustrates a method 1700 for treating a patient using a multi-electrode pad. At 1702, the flexible electrode pad (e.g., multi-electrode pad 102) can be positioned on a patient's skin. The position can be on or near a target tissue in the patient's body. The target tissue can correspond to a portion of a spinal cord, one or more spinal nerves, or one or more peripheral nerves. At 1704, one or more electrical stimulations (e.g., configured by controller 106 and/or stimulator 104) can be applied to the patient via one or more electrodes (e.g., of the plurality of electrodes 122) on the flexible electrode pad (e.g., multi-electrode pad 102).

From the above description, those skilled in the art will perceive improvements, changes, and modifications. Such improvements, changes and modifications are within the skill of one in the art and are intended to be covered by the appended claims.

Further examples according to the present disclosure are described below in numbered clauses:
1. An electrode pad configured to adhere to a patient's skin and provide transcutaneous stimulation to a portion of tissue, the electrode pad comprising:
   a flexible substrate;
   a plurality of electrodes arranged in an array on or within the flexible substrate, wherein each of the plurality of electrodes is configured to apply a stimulation waveform; and
   conductive traces applied to the flexible substrate and coupled to each of the plurality of electrodes such that each of the plurality of electrodes is independently addressable through a single external cable.
2. The electrode pad of clause 1, further comprising an adhesive layer that covers at least a portion of the flexible substrate and the conductive traces without covering the plurality of electrodes.
3. The electrode pad of clause 1, wherein the portion of tissue comprises at least a portion of a spinal cord, one or more spinal nerves, or one or more peripheral nerves.
4. The electrode pad of clause 1, wherein the flexible substrate is at least partially skeletonized and/or comprises one or more slits or cutouts configured to allow at least a portion of the array to conform to a surface.
5. The electrode pad of clause 1, wherein at least one of the plurality of electrodes comprises a pattern designed to spread electric field evenly or to reduce impedance between the at least one of the plurality of electrodes and tissue the at least one of the plurality of electrodes contacts.
6. The electrode pad of clause 1, further comprising a connector to couple the plurality of electrodes to the single cable.
7. The electrode pad of clause 1, further comprising one or more components configured to measure a bending moment of the array, an orientation angle of the array, a linear acceleration of the array, and/or a radial acceleration of the array.
8. The electrode pad of clause 1, wherein one or more of the plurality of electrodes is functionalized to detect a chemical and/or biological signature of inflammation and/or an allergic reaction in tissue in contact with the one or more of the plurality of electrodes.
9. The electrode pad of clause 1 further comprising one or more light emitters arranged on or in the flexible substrate, each configured to provide photobiomodulation therapy to surrounding tissue.
10. The electrode pad of clause 1, wherein two or more of the plurality of electrodes are configured to form a virtual electrode with a synchronized stimulation timing.
11. A system comprising:
   at least one flexible electrode pad, each comprising a plurality of electrodes arranged on or within a flexible substrate in an array and connected by conductive traces such that each of the plurality of electrodes is independently addressable through a single external cable;
   a stimulator connected to the flexible electrode pad through the single external cable configured to provide a stimulation to at least a portion of the plurality of electrodes based on addresses associated with the at least the portion of the plurality of electrodes; and
   a controller coupled to the stimulator comprising a processor configured to select the portion of the plurality of electrodes and to alter one or more parameters of the stimulation for the portion of the plurality of electrodes based on a user input.
12. The system of clause 10, wherein the processor is configured to alter the one or more parameters of the stimulation based on an algorithm intended to stimulate with a high focality and intensity.
13. The system of clause 10, further comprising one or more ground electrodes connected to the stimulator.
14. The system of clause 10, further comprising one or more sensors configured to be placed on or around a patient's body, wherein the sensor comprises one or more of an inertial measurement unit, a bend sensor, an electromyogram (EMG) sensor, and a near-infrared spectrum (NIRS) sensor.
15. The system of clause 10, further comprising a flexible hub configured to be placed between the plurality of electrodes and the single external cable to route electrical stimulation to an appropriate at least one flexible electrode pad.
16. A method comprising:
   printing a first conductive layer, comprising a plurality of electrodes, traces, and connections, on a flexible substrate using conductive ink;
   printing a dielectric layer covering a portion of the conductive ink to insulate the traces, but leave connections and electrodes exposed;
   placing an adhesive layer on top of the dielectric layer; and
   cutting the substrate into a shape of an electrode pad,
   wherein the plurality of electrodes is arranged in an array.
17. The method of clause 16, further comprising placing a connector on the substrate to form electrical connections with the traces.
18. The method of clause 16, further comprising cutting the flexible substrate in a skeletonized pattern such that one or more electrodes in the array are able to move independently from others of the plurality of electrodes.
19. The method of clause 16, further comprising placing an isolated hydrogel section at least on top of each electrode.
20. The method of clause 16, further comprising placing a foam layer between the substrate and the adhesive layer.
21. The method of clause 16, further comprising printing a second conductive layer after printing the first conductive layer to form three dimensional patterns on at least one of the plurality of electrodes.

## Claims

1. An electrode pad configured to adhere to a patient's skin and provide transcutaneous stimulation to a portion of tissue, the electrode pad comprising:
a skeletonized flexible substrate (120), wherein the skeletonized flexible substrate comprises a plurality of peninsulas formed by one or more slits or cutouts in the flexible substrate;
a plurality of electrodes (122) arranged in an array, wherein each of the plurality of electrodes is configured to apply a stimulation waveform and wherein at least one electrode of the plurality of electrodes is on or within each of the plurality of peninsulas;
conductive traces (124) applied to the skeletonized flexible substrate and coupled to each of the plurality of electrodes such that each of the plurality of electrodes is independently addressable through a single external cable; and
a hub connected to the single external cable.

2. The electrode pad of claim 1, further comprising a cryptographic identification chip configured to communicate with an external device,
wherein the cryptographic identification chip is configured to communicate with the external device via a wired connection.

3. The electrode pad of claim 2, wherein the wired connection comprises the single external cable.

4. The electrode pad of claim 3, wherein the single external cable is also used to provide the stimulation waveform to the plurality of electrodes.

5. The electrode pad of claim 2, further comprising additional conductive traces to the cryptographic identification chip.

6. The electrode pad of claim 2, wherein the cryptographic identification chip is configured to verify that the electrode pad is genuine before providing the stimulation.

7. The electrode pad of claim 2, wherein the cryptographic identification chip is configured to generate an alert when the pad is expired or had been previously used.

8. The electrode pad of claim 2, wherein the cryptographic identification chip is configured to store calibration information about sizes and/or positions of the electrodes.

9. The electrode pad of claim 1, wherein electrodes on or within each of the plurality of peninsulas are configured to move independently of electrodes on or within other peninsulas of the plurality of peninsulas.

10. The electrode pad of claim 1, wherein the stimulation is used to improve autonomic function in a patient with at least one impaired autonomic function.

11. The electrode pad of claim 1, wherein the stimulation is used to treat symptoms of spinal cord injury, cerebral palsy, Parkinson's disease, essential tremor, stroke, amyotrophic lateral sclerosis (ALS), a disease that involves pain, or a disease that involves impaired communication between the brain and body.

12. The electrode pad of claim 1, wherein the electrode pad is connector-less.

13. A system (100) comprising:
at least one flexible electrode pad (102), and each of the at least one flexible electrode pad embodied as two or more peninsulas separated by slits or cutouts in a flexible substrate that facilitate movement of the two or more peninsulas respective to each other, wherein each of the two or more peninsulas comprises a plurality of electrodes (122) arranged on or within a flexible substrate (120) in an array and connected by conductive traces (124) such that each of the plurality of electrodes is independently addressable through the single external cable, wherein the two or more peninsulas are able to conform to a surface;
a stimulator (104) connected to the flexible electrode pad through the single external cable configured to provide a stimulation to at least a portion of the plurality of electrodes based on addresses associated with the at least the portion of the plurality of electrodes; and
a controller (106) coupled to the stimulator comprising a processor configured to select the portion of the plurality of electrodes and to alter one or more parameters of the stimulation for the portion of the plurality of electrodes based on a user input.

14. The system of claim 13, further comprising a cryptographic identification chip configured to communicate with an external device.

15. The system of claim 13, wherein the stimulation is used to treat symptoms of spinal cord injury, cerebral palsy, Parkinson's disease, essential tremor, stroke, amyotrophic lateral sclerosis (ALS), a disease that involves pain, or a disease that involves impaired communication between the brain and body.
